(19) Europäisches Patentamt
European Patent Office
Office européen des brevets

(11) Veröffentlichungsnummer: **0 168 701**
**B1**

(12) # EUROPÄISCHE PATENTSCHRIFT

(45) Veröffentlichungstag der Patentschrift: **02.11.88**

(21) Anmeldenummer: **85108093.7**

(22) Anmeldetag: **29.06.85**

(51) Int. Cl.[4]: **C 07 C 121/46,** C 07 C 69/753, C 07 C 69/03, C 07 C 49/313, C 07 C 43/184, C 09 K 19/30

(54) **Tercyclohexyle.**

(30) Priorität: **14.07.84 DE 3426035**
**18.07.84 DE 3426522**

(43) Veröffentlichungstag der Anmeldung:
**22.01.86 Patentblatt 86/4**

(45) Bekanntmachung des Hinweises auf die Patenterteilung:
**02.11.88 Patentblatt 88/44**

(84) Benannte Vertragsstaaten:
**DE FR IT**

(56) Entgegenhaltungen:
**EP-A-0 090 671**
**EP-A-0 120 627**

**CHEMICAL ABSTRACTS, Band 102, Nr. 5, 4. Februar 1985, Seite 545, Spalte 1, Zusammenfassungsnr. 45632y, Columbus, Ohio, US; CHISSO CORP. "Tricyclic carboxylic acid ester derivatives"**
**PATENT ABSTRACTS OF JAPAN, Band 9, Nr. 1 (C-259) 1724, 5. Januar 1985**

(73) Patentinhaber: **Merck Patent Gesellschaft mit beschränkter Haftung, Frankfurter Strasse 250, D-6100 Darmstadt (DE)**

(72) Erfinder: **Sucrow, Wolfgang, Prof. Dr., Hüfferweg 13, D-4790 Paderborn (DE)**
Erfinder: **Wolter, Herbert, Am Ziegenberg 5, D-4790 Paderborn- Barkhausen (DE)**
Erfinder: **Eidenschink, Rudolf, Dr., Kornblumenstrasse 1, D-6115 Münster (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 168 701 B1

LIBER, STOCKHOLM 1988

**Beschreibung**

Die Erfindung betrifft für den Vertragsstaat DE neue Tercyclohexyle der Formel I

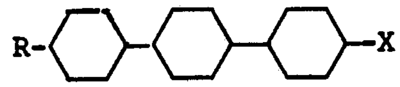

I

worin

R eine Alkylgruppe mit 1 - 12 C-Atomen, worin eine oder zwei nicht benachbarte $CH_2$-Gruppen durch 0 ersetzt sein können

X $-COOR^1$, $-OOCR^1$, $-COR^1$ oder $R^1$ und

$R^1$ eine Alkylgruppe mit 1 - 12 C-Atomen bedeuten, mit der Maßgabe, daß im Falle $X = R^1$ in der Alkylgruppe R mindestens eine $CH_2$-Gruppe durch 0 ersetzt ist und für die Vertragsstaaten FR und IT Verbindungen der Formel I worin R und $R^1$ die oben angegebene Bedeutung haben und X -CN, $-COOR^1$, $-OOCR^1$, $-COR^1$ oder $R^1$ beduetet.

Die Verbindungen der Formel I können wie ähnliche, z. B. aus der DE-OS-2 702 598 bekannte Verbindungen als Komponenten flüssigkristalliner Dielektrika verwendet werden, insbesondere für Displays, die auf dem Prinzip der verdrillten Zelle, dem Guest-Host-Effekt, dem Effekt der Deformation aufgerichteter Phasen oder dem Effekt der dynamischen Streuung beruhen.

Der Erfindung lag die Aufgabe zugrunde, neue stabile flüssigkristalline oder mesogene Verbindungen aufzufinden, die als Komponenten flüssigkristalliner Dielektrika geeignet sind.

Es wurde gefunden, daß die Verbindungen der Formel I als Komponenten flüssigkristalliner Dielektrika vorzüglich geeignet sind. Insbesondere sind mit ihrer Hilfe stabile flüssigkristalline Phasen mit relativ geringer optischer Anisotropie und mit hohem nematischen Charakter herstellbar, die sich in elektrooptischen Anzeigeelementen nach dem Prinzip der verdrillten Zelle und/oder dem Guest-Host-Effekt durch eine besonders günstige Winkelabhängigkeit des Kontrastes auszeichnen.

Mit der Bereitstellung der Verbindungen der Formel I wird außerdem ganz allgemein die Palette der flüssigkristallinen Substanzen, die sich unter verschiedenen anwendungstechnischen Gesichtspunkten zur Herstellung nematischer Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Phasen zum überwiegenden Teil zusammengesetzt sind, es können aber auch Verbindungen der Formel I flüssigkristallinen Basismaterialien aus anderen Verbindungsklassen zugesetzt werden, um beispielsweise die Winkelabhängigkeit des Kontrastes und/oder die optische Anisotropie einer solchen Phase zu beeinflussen. Die Verbindungen der Formel I eignen sich ferner als Zwischenprodukte zur Herstellung anderer Substanzen, die sich als Bestandteile flüssigkristalliner Dielektrika verwenden lassen.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie sehr stabil.

Gegenstand der Erfindung sind somit die Verbindungen der Formel I sowie ein Verfahren zu ihrer Herstellung, dadurch gekennzeichnet, daß man zur Herstellung von Carbonitrilen der Formel I (X = CN) die entsprechenden Tercyclohexylcarbonsäuren (X = COOH) oder eines ihrer reaktionsfähigen Derivate in die entsprechenden Säureamide überführt und diese dehydratisiert, oder aber ein entsprechendes Säurechlorid mit Sulfamid einsetzt, oder daß man zur Herstellung von Carbonsäureestern der Formel I ($X = -COOR^1$) die entsprechenden Carbonsäureverbindungen oder eines ihrer reaktionsfähigen Derivate mit einem entsprechenden Alkohol oder einem seiner reaktionsfähigen Derivate umsetzt, oder daß man zur Herstellung von Acylverbindungen der Formel I ($X = -COR_1$) die entsprechenden Carbonsäureverbindungen oder eines ihrer reaktionsfähigen Derivate in die entsprechenden Nitrile überführt und diese mit einer entsprechenden Grignard-Verbindung umsetzt, oder daß man zur Herstellung der Alkoxy-/Alkyl-Verbindungen ($X = R^1$) die entsprechenden Carbonsäureverbindungen oder eines ihrer reaktionsfähigen Derivate in die entsprechenden Ketoverbindungen ($X = -COR^1$) überführt und diese reduziert, oder daß man eine Verbindung, die sonst der Formel I entspricht, aber an Stelle von H-Atomen eine oder mehrere reduzierbare Gruppen und/oder C-C-Bindungen enthält, mit einem reduzierenden Mittel behandelt, oder daß man zur Herstellung von Tercyclohexylen der Formel I'

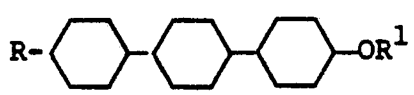

I'

worin

R eine Alkylgruppe mit 1 - 12 C-Atomen, worin eine oder zwei nicht benachbarte $CH_2$-Gruppen durch 0 ersetzt sein können, und

$R^1$ eine Alkylgruppe mit 1 - 12 C-Atomen bedeutet, dadurch gekennzeichnet, daß man die entsprechenden Tercyclohexanole ($R^1$ = H) oder eines ihrer reaktionsfähigen Derivate verethert,

oder daß man zur von Alkanoyloxyderivaten der Formel I (X = -OOCR$^1$) die entsprechenden Tercyclohexanole (X = OH) oder eines ihrer reaktionsfähigen Derivate mit einer entsprechenden Carbonsäure oder einem ihrer reaktionsfähigen Derivate umsetzt.

Weiterhin ist Gegenstand der Erfindung die Verwendung der Verbindungen der Formel I als Komponenten flüssigkristalliner Phasen. Gegenstand der Erfindung sind ferner flüssigkristalline Phasen mit einem Gehalt an mindestens einer Verbindung der Formel I sowie Flüssigkristallanzeigeelemente, insbesondere elektrooptische Anzeigeelemente, die derartige Phasen enthalten.

Der Einfachheit halber bedeutet im folgenden "Cyc" eine 1,4-Cyclohexylengruppe.

In den Verbindungen der Formel I sind diejenigen Stereoisomeren bevorzugt, worin alle drei 1,4-Cyclohexylengruppen trans-ständig in 1,4-Stellung substituiert sind.

Die Verbindungen der Formel I umfassen dementsprechend bevorzugte Verbindungen der Teilformeln Ia bis If

| | |
|---|---|
| R-Cyc-Cyc-Cyc-CN | Ia |
| R-Cyc-Cyc-Cyc-$R^1$ | Ib |
| R-Cyc-Cyc-Cyc-$COOR^1$ | Ic |
| R-Cyc-Cyc-Cyc-CO-$R^1$ | Id |
| R-Cyc-Cyc-Cyc-$OOCR^1$ | Ie |
| $R^1$-Cyc-Cyc-Cyc-$CH_2O$Alkyl | If |

Vorzugsweise werden Verbindungen der Teilformeln Ia - Ic und Ie, insbesondere Ia, Ib und Ie verwendet. Besonders bevorzugt sind Ib und Ie.

Weiterhin bevorzugt sind die Verbindungen der Teilformeln Ia und Ic mit axialer -CN bzw. -$COOR^1$-Gruppe, d. h. die Verbindungen der Teilformeln Iaa und Ica:

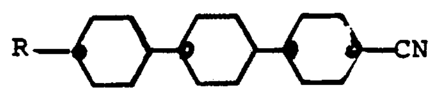

Iaa

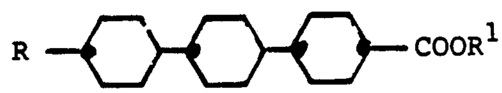

Ica

In den Verbindungen der vor- und nachstehenden Formeln können die Alkylreste R, worin auch eine ("Alkoxy" bzw. "Oxaalkyl") oder zwei ("Alkoxyalkoxy" bzw. "Dioxaalkyl") nicht benachbarte $CH_2$-Gruppen durch O-Atome ersetzt sein können, geradkettig oder verzweigt sein. Vorzugsweise sind sie geradkettig, haben 2, 3, 4, 5, 6, 7, 8, 9 oder 10 C-Atome und bedeuten demnach bevorzugt Ethyl, Propyl, Butyl, Pentyl, Hexyl, Heptyl, Octyl, Nonyl, Decyl, Ethoxy, Propoxy, Butoxy, Pentoxy, Hexoxy, Heptoxy, Octoxy, Nonoxy, Decoxy, 2-Oxapropyl (= Methoxymethyl), 2-(=Ethoxymethyl) oder 3-Oxabutyl (= 2-Methoxymethyl), 2-, 3- oder 4-Oxapentyl 2-, 3-, 4- oder 5-Oxahexyl, 2-, 3-, 4-, 5- oder 6-Oxaheptyl, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl, ferner Methyl, Undecyl, Dodecyl, Methoxy, Undecoxy, Dodecoxy, 2-, 3-, 4-, 5-, 6-, 7-, 8-, 9- oder 10-Oxaundecyl, 2-, 3-, 4-, 5-, 6-, 7-, 8-, 9-, 10- oder 11-Oxadodecyl, 1,3-Dioxabutyl (= Methoxymethoxy), 1,3-, 1,4- oder 2,4-Dioxapentyl, 1,3-, 1,4-, 1,5-, 2,4-, 2,5- oder 3,5-Dioxahexyl, 1,3-, 1,4-, 1,5-, 1,6-, 2,4-, 2,5-, 2,6-, 3,5-, 3,6- oder 4,6-Dioxaheptyl 1,3-, 1,4-, 1,5-, 1,6-, 1,7-, 2,4-, 2,5-, 2,6-, 2,7-, 3,5-, 3,6-, 3,7-, 4,6-, 4,7- oder 5,7-Dioxaoctyl, 1,3-, 1,4-, 1,5-, 1,6-, 1,7-, 1,8-, 2,4-, 2,5-, 2,6-, 2,7-, 2,8-, 3,5-, 3,6-, 3,7-, 3,8-, 4,6-, 4,7-, 4,8-, 5,7- oder 5,8-Dioxanonyl, 1,3-, 1,4-, 1,5-, 1,6-, 1,7-, 1,8-, 1,9-, 2,4-, 2,5-, 2,6-, 2,7-, 2,8-, 2,9-, 3,5-, 3,6-, 3,7-, 3,8-, 3,9-, 4,6-, 4,7-, 4,8-, 4,9-, 5,7-, 5,8- oder 5,9-Dioxadecyl.

R ist im Falle X = -CN, -$COOR^1$, $OOCR^1$ oder -$COR^1$ vorzugsweise Alkyl oder Alkoxy, insbesondere n-Alkyl; im Falle X = $R^1$ vorzugsweise n-Alkoxy oder n-Oxaalkyl, insbesondere bevorzugt n-Alkoxy.

Verbindungen der Formeln I sowie Ia bis If mit verzweigten Flügelgruppen R bzw. $R^1$ können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Verzweigte Gruppen dieser Art enthalten in der Regel nicht mehr als eine Kettenverzweigung. Bevorzugte verzweigte Reste R und $R^1$ sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy, 2-Oxa-3-methylbutyl, 3-Oxa-4-methylpentyl. Derartige Verbindungen eignen sich als Komponenten ferroelektrischer Flüssigkristallphasen.

Unter den Verbindungen der Formeln I sowie Ia bis If sind diejenigen bevorzugt, in denen mindestens einer der darin enthaltenen Reste eine der angegebenen bevorzugten Bedeutungen hat.

Besonders bevorzugte Gruppen von Verbindungen entsprechen den Formeln Ig bis Il:

| | |
|---|---|
| Alkyl-Cyc-Cyc-Cyc-CN | Ig |
| Alkoxy-Cyc-Cyc-Cyc-CN | Ih |
| Alkoxy-Cyc-Cyc-Cyc-Alkyl | Ii |
| Alkyl-Cyc-Cyc-Cyc-COOAlkyl | Ij |

Alkyl-Cyc-Cyc-Cyc-OOCAlkyl Ik
Alkyl-Cyc-Cyc-Cyc-$CH_2O$Alkyl Il

worin die Alkyl- und Alkoxygruppen 1 - 10, vorzugsweise 2 - 8 C-Atome besitzen, vorzugsweise geradkettig sind und die beiden Alkylgruppen in Ih gleich oder voneinander verschieden sein können.

Ferner bevorzugte Verbindungen der Formel I entsprechen den Formeln Im bis Io:

n-Alkyl-Cyc-Cyc-Cyc-OOC-$CH_2CH_2CH_3$ Im
n-Alkyl-Cyc-Cyc-Cyc-O-$CH_2CH_3$ In
n-Alkyl-Cyc-Cyc-Cyc-O-$CH_2CH_2CH_2CH_3$ Io

worin die n-Alkylgruppen 2 - 8 C-Atome besitzen.

Die Verbindungen der Formel I können nach an sich bekannten Methoden hergestellt werden, wie sie in der Literatur (z. B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt. Als Ausgangsstoffe verwendet man vorzugsweise die entsprechenden Bicyclohexylcyclohexancarbonsäuren, deren Herstellung in der EP-90 671 als nicht näher charakterisierte Zwischenstufen bei der Synthese von flüssigkristallinen Vierringsystemen beschrieben wurde.

Neben den entsprechenden freien Tercyclohexylcarbonsäuren eignen sich auch ihre reaktionsfähigen Derivate.

Als reaktionsfähige Derivate der genannten Carbonsäuren eignen sich insbesondere die Säurehalogenide, vor allem die Chloride und Bromide, ferner die Anhydride, Azide oder Ester, insbesondere Alkylester mit 1 - 4 C-Atomen in der Alkylgruppe.

Als Ausgangsstoffe verwendet man weiterhin vorzugsweise die entsprechenden Bicyclohexylcyclohexanole, deren Herstellung in der EP-90 671 als nicht näher charakterisierte Zwischenstufe bei der Synthese von flüssigkristallinen Vierringsystemen beschrieben wurde.

Neben den entsprechenden freien Tercyclohexanolen eignen sich auch ihre reaktionsfähige Derivate.

Als reaktionsfähige Derivate der genannten Alkohole kommen insbesondere die entsprechenden Metallalkoholate der Formel $R^1OM$ in Betracht, worin M ein Äquivalent eines Metalls, vorzugsweise eines Alkalimetalls wie Na oder K, bedeutet.

Zur Herstellung der Nitrile der Formel I (X = CN) können entsprechende Säureamide, in denen an Stelle der CN-Gruppe eine $CONH_2$-Gruppe steht, dehydratisiert werden. Die Amide sind z. B. aus den entsprechenden Säurehalogeniden durch Umsetzung mit Ammoniak erhältlich. Die entsprechenden Säurehalogenide lassen sich wiederum auf bekannte Weise aus den entsprechenden Carbonsäuren, z. B. mit Thionylchlorid, herstellen. Als wasserabspaltende Mittel zur Dehydratisierung der Amide eignen sich beispielsweise anorganische Säurechloride wie $SOCl_2$, $PCl_3$, $PCl_5$, $POCl_3$, $SO_2Cl_2$, $COCl_2$, ferner $P_2O_5$, $AlCl_3$ (z. B. als Doppelverbindung mit NaCl), aromatische Sulfonsäuren und Sulfonsäurehalogenide. Man kann dabei in Gegenwart oder Abwesenheit eines inerten Lösungsmittels bei Temperaturen zwischen etwa 0° und 150° arbeiten; als Lösungsmittel kommen z. B. Basen wie Pyridin oder Triethylamin, aromatische Kohlenwasserstoffe wie Benzol, Toluol oder Xylol oder Amide wie DMF in Betracht.

Zur Herstellung der Nitrile der Formel I kann man auch entsprechende Säurehalogenide, vorzugsweise die Chloride, mit Sulfamid umsetzen, zweckmäßig in einem inerten Lösungsmittel wie Tetramethylensulfon bei Temperaturen zwischen etwa 80° und 150°, vorzugsweise bei 120°. Nach üblicher Aufarbeitung kann man direkt die Nitrile isolieren.

Zur Herstellung der Ester der Formel I (X = -OOC$R^1$) wird vorzugsweise ein entsprechendes Tercyclohexanol oder eines seiner reaktionsfähigen Derivate mit einer entsprechenden Carbonsäure oder einem ihrer reaktionsfähigen Derivate umgesetzt.

Zur Herstellung der Ester der Formel I (X = -COO$R^1$) wird vorzugsweise eine entsprechende Carbonsäure oder eines ihrer reaktionsfähigen Derivate mit einem entsprechenden Alkohol oder einem seiner reaktionsfähigen Derivate umgesetzt.

Als reaktionsfähige Derivate der genannten Alkohole kommen insbesondere die entsprechenden Metallalkoholate der Formel $R^1OM$ in Betracht, worin M ein Äquivalent eines Metalls, vorzugsweise eines Alkalimetalls wie Na oder K, bedeutet.

Als reaktionsfähige Derivate der genannten Carbonsäuren eignen sich insbesondere die Säurehalogenide, vor allem die Chloride und Bromide, ferner die Anhydride, Azide oder Ester, insbesondere Alkylester mit 1 - 4 C-Atomen in der Alkylgruppe.

Die Veresterung wird vorteilhaft in Gegenwart eines inerten Lösungsmittels durchgeführt. Gut geeignet sind insbesondere Ether wie Diethylether, Di-n-butylether, THF, Dioxan oder Anisol, Ketone wie Aceton, Butanon oder Cyclohexanon, Amide wie DMF oder Phosphorsäurehexamethyltriamid, Kohlenwasserstoffe wie Benzol, Toluol oder Xylol, Halogenkohlenwasserstoffe wie Tetrachlorkohlenstoff oder Tetrachlorethylen und Sulfoxide wie Dimethylsulfoxid oder Sulfolan. Mit Wasser nicht mischbare Lösungsmittel können gleichzeitig vorteilhaft

zum azeotropen Abdestillieren des bei der Veresterung gebildeten Wassers verwendet werden. Gelegentlich kann auch ein Überschuß einer organischen Base, z. B. Pyridin, Chinolin oder Triethylamin als Lösungsmittel für die Veresterung angewandt werden. Die Veresterung kann auch in Abwesenheit eines Lösungsmittels, z. B. durch einfaches Erhitzen der Komponenten in Gegenwart von Natriumacetat, durchgeführt werden. Die Reaktionstemperatur liegt gewöhnlich zwischen -50° und +250°, vorzugsweise zwischen -20° und +80°. Bei diesen Temperaturen sind die Veresterungsreaktionen in der Regel nach 15 Minuten bis 48 Stunden beendet.

Im einzelnen hängen die Reaktionsbedindungen für die Veresterung weitgehend von der Natur der verwendeten Ausgangsstoffe ab. So wird eine freie Carbonsäure mit einem freien Alkohol in der Regel in Gegenwart einer starken Säure, beispielsweise einer Mineralsäure wie Salzsäure oder Schwefelsäure, umgesetzt. Eine bevorzugte Reaktionsweise ist die Umsetzung eines Säureanhydrids oder insbesondere eines Säurechlorids mit einem Alkohol, vorzugsweise in einem basischen Milieu, wobei als Basen insbesondere Alkalimetallcarbonate bzw. -hydrogencarbonate wie Natriumcarbonat, Natriumhydrogencarbonat, Kaliumcarbonat oder Kaliumhydrogencarbonat, Alkalimetallacetate wie Natrium- oder Kaliumacetat, Erdalkalimetallhydroxide wie Calciumhydroxid oder organische Basen wie Triethylamin, Pyridin, Lutidin, Kollidin oder Chinolin von Bedeutung sind. Eine weitere bevorzugte Ausführungsform der Veresterung besteht darin, daß man den Alkohol zunächst in das Natrium- oder Kaliumalkoholat überführt, z. B. durch Behandlung mit ethanolischer Natron- oder Kalilauge, dieses isoliert und zusammen mit Natriumhydrogencarbonat oder Kaliumcarbonat unter Rühren in Aceton oder Diethylether suspendiert und diese Suspension mit einer Lösung des Säurechlorids oder Anhydrids in Diethylether, Aceton oder DMF versetzt, zweckmäßig bei Temperature zwischen etwa -25° und +20°.

Zur Herstellung der Acylverbindungen der Formel I (X = $-CO-R^1$) aus den entsprechenden Carbonsäuren wird zunächst, wie beschrieben, das entsprechende Nitril hergestellt. Letzteres wird vorzugsweise mit einer Grignardverbindung der allgemeinen Formel $R^1MHal$, worin M Metall, vorzugsweise Magnesium und Hal Halogen, vorzugsweise Bromid bedeutet, in an sich bekannter Weise umgesetzt und anschließend hydrolysiert. Die Grignard-Verbindung wird in bekannter Weise mit Magnesium und dem entsprechenden Alkylhalogenid $R^1Hal$, vorzugsweise $R^1Br$, in einem Ether, vorzugsweise Diethylether oder Tetrahydrofuran unter für derartige Reaktionen üblichen Bedingungen hergestellt.

Zur Herstellung der Alkoxy-Alkyl-Verbindungen der Formel I (X = $R^1$) werden vorzugsweise die entsprechenden Ketone (X = $-CO-R^1$) nach den Methoden von Clemmensen (mit Zink, amalgamiertem Zink oder Zinn und Salzsäure, zweckmäßig in wäßrig-alkoholischer Lösung oder in heterogener Phase mit Wasser/Toluol bei Temperaturen zwischen etwa 80° und 120°) oder Wolff-Kishner (mit Hydrazin, zweckmäßig in Gegenwart von Alkali wie KOH oder NaOH in einem hochsiedenden Lösungsmittel wie Diethylenglykol oder Triethylenglykol bei Temperaturen zwischen etwa 100° und 200°) zu den entsprechenden Verbindungen der Formel I reduziert.

Die Verbindungen der Formel I können auch hergestellt werden, indem man Verbindungen, die sonst der Formel I entsprechen, aber an Stelle von H-Atomen eine oder mehrere reduzierbare Gruppen und/oder C-C-Bindungen enthalten, reduziert.

Als reduzierbare Gruppen kommen vorzugsweise Carbonylgruppen in Betracht, insbesondere Ketogruppen, ferner z. B. freie oder veresterte Hydroxygruppen. Bevorzugte Ausgangsstoffe für die Reduktion entsprechen der Formel I, können aber an Stelle eines Cyclohexanrings einen Cyclohexenring oder Cyclohexanonring und/oder an Stelle einer $-CH_2CH_2-$Gruppe eine -CO-Gruppe und/oder an Stelle einer $-CH_2-$Gruppe eine -CO-Gruppe und/oder an Stelle eines H-Atomes eine freie oder eine funktionell (z. B. in Form ihres p-Toluolsulfonats) abgewandelte OH-Gruppe enthalten.

Die Reduktion kann z. B. erfolgen durch katalytische Hydrierung bei Temperaturen zwischen etwa 0° und etwa 200° sowie Drucken zwischen etwa 1 und 200 bar in einem inerten Lösungsmittel, z. B. einem Alkohol wie Methanol, Ethanol oder Isopropanol, einem Ether wie Tetrahydrofuran (THF) oder Dioxan, einem Ester wie Ethylacetat, einer Carbonsäure wie Essigsäure oder einem Kohlenwasserstoff wie Cyclohexan. Als Katalysatoren eignen sich zweckmäßig Edelmetalle wie Pt oder Pd, die in Form von Oxiden (z. B. $PtO_2$, PdO), auf einem Träger (z. B. Pd auf Kohle, Calciumcarbonat oder Strontiumcarbonat) oder in feinverteilter Form eingesetzt werden können.

Zur Herstellung der Ether der Formel I' wird vorzugsweise ein entsprechendes Tercyclohexanol oder eines seiner reaktionsfähigen Derivate mit einem entsprechenden Alkylhalogenid, -sulfonat oder Dialkylsulfat umgesetzt, zweckmäßig in einem inerten Lösungsmittel wie Aceton, 1,2-Dimethoxyethan, DMF oder Dimethylsulfoxid oder auch einem Überschuß an wäßriger oder wäßrig-alkoholischer NaOH oder KOH bei Temperaturen zwischen etwa 20° und 100°.

Zweckmäßig wird die Hydroxyverbindung zuvor in ein entsprechendes Metallderivat, z. B. durch Behandeln mit NaH, $NaNH_2$, NaOH, KOH, $Na_2CO_3$ oder $K_2CO_3$ in das entsprechende Alkalimetallalkoholat überführt.

Die erfindungsgemäßen Dielektrika bestehen aus 2 bis 25, vorzugsweise 3 bis 15 Komponenten, darunter mindestens einer Verbindung der Formel I. Die anderen Bestandteile werden vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl- oder Cyclohexylbenzoate, Cyclohexancarbon-säurephenyl- oder -cyclohexyl-ester, Phenylcyclohexane, Cyclohexylbiphenyle, Cyclohexylcyclohexane, Cyclohexylnaphthaline, 1,4-Bis-cyclohexylbenzole, 4,4'-Bis-cyclohexylbiphenyle, Phenyl- oder Cyclohexylpyrimidine, Phenyl- oder Cyclohexyldioxane, 1,2-Bis-cyclohexylethane, 1-Cyclohexyl-2-phenylethane, 1,2-Bis-phenylethane, gegebenenfalls halogenierten Stilbene, Benzylphenylether, Tolane und

substituierten Zimtsäuren.

Die wichtigsten als Bestandteile derartiger flüssigkristalliner Dielektrika in Frage kommenden Verbindungen lassen sich durch die Formel II charakterisieren.

R'-L-G-E-R'' II

worin L und E je ein carbo- oder heterocyclisches Ringsystem aus der aus unsubstituierte oder durch ein oder zwei F- und/oder Cl-Atome und/oder $CH_3$-Gruppen und/oder CN-Gruppen substituierten 1,4-Phenylen- und trans-1,4-Cyclohexylringen, 4,4'-disubstituierten Biphenyl-, Phenylcyclohexan- und Cyclohexylcyclohexansystemen, 2,5-disubstituierten Pyrimidin- und 1,3-Dioxanringen, 2,6-disubstituiertem Naphthalin, Di- und Tetrahydronaphthalin, Chinazolin und Tetrahydrochinazolin gebildeten Gruppe,

| | |
|---|---|
| -CH=CH- | -N(O)=N- |
| -CH=CY- | -CH=N(O)- |
| -C≡C- | $-CH_2-CH_2-$ |
| -CO-O- | $-CH_2-O-$ |
| -CO-S- | $-CH_2-S-$ |
| -CH=N- | -COO-Phe-COO- |

oder eine C-C-Einfachbindung, Y Halogen, vorzugsweise Chlor, oder -CN, und R' und R'' Alkyl, Alkoxy, Alkanoyloxy oder Alkoxycarbonyloxy mit bis zu 18, vorzugsweise bis zu 8 Kohlenstoffatomen, oder einer dieser Reste auch CN, NC, $NO_2$, $CF_3$, F, Cl oder Br bedeuten.

Bei den meisten dieser Verbindungen sind R' und R'' voneinander verschieden, wobei einer dieser Reste meist eine Alkyl- oder Alkoxygruppe ist. Aber auch andere Varianten der vorgesehenen Substituenten sind gebräuchlich. Viele solcher Substanzen oder auch Gemische davon sind im Handel erhältlich. Alle diese Substanzen sind nach literaturbekannten Methoden herstellbar.

Die erfindungsgemäßen Dielektrika enthalten in der Regel mindestens 30, vorzugsweise 50 - 99, insbesondere 60 - 98 Gewichtsprozent der Verbindungen der Formel I und II. Hiervon entfallen bevorzugt mindestens 5 Gewichtsprozent meist auch 10 - 40 Gewichtsprozent auf eine oder mehrere Verbindungen der Formel I. Jedoch werden von der Erfindung auch solche flüssigkristallinen Dielektrika umfaßt, denen beispielsweise zu Dotierungszwecken nur weniger als 5 Gewichtsprozent zum Beispiel 0,1 bis 3 Gewichtsprozent einer oder mehrerer Verbindungen der Formel I zugesetzt worden sind. Andererseits können die Verbindungen der Formel I bis zu 60 Gewichtsprozent der erfindungsgemäßen Dielektrika ausmachen. Vorzugsweise enthalten die flüssigkristallinen Dielektrika nach der Erfindung 10 bis 30 Gewichtsprozent einer oder mehrerer Verbindungen der Formel I.

Die Herstellung der erfindungsgemäßen Dielektrika erfolgt in an sich üblicher Weise. In der Regel werden die Komponenten ineinander gelöst, zweckmäßig bei erhöhter Temperatur. Durch geeignete Zusätze können die flüssigkristallinen Dielektrika nach der Erfindung so modifiziert werden, daß sie in allen bisher bekannt gewordenen Arten von Flüssigkristallanzeigeelementen verwendet werden können.

Derartige Zusätze sind dem Fachmann bekannt und in der Literatur ausführlich beschrieben. Beispielsweise können Leitsalze, vorzugsweise Ethyl-dimethyl-dodecyl-ammonium-4-hexyloxybenzoat, Tetrabutylammoniumtetraphenylboranat oder Komplexsalze von Kronenethern (vgl. z. B. I. Haller et al., Mol. Cryst. Liq. Cryst. Band 24, Seiten 249-258 (1973)) zur Verbesserung der Leitfähigkeit, dichroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden. Derartige Substanzen sind z. B. in den DE-OS-2 209 127, 2 240 864, 2 321 632, 2 338 281, 2 450 088, 2 637 430, 2 853 728 und 2 902 177 beschrieben.

Die folgenden Beispiele sollen die Erfindung erläutern. (Schmp. = Schmelzpunkt, Klp. = Klärpunkt.)

**Beispiele 1 - 18**

**Beispiel 1**

a) 38 g 4-Bicyclohexylyl-cyclohexylcarbonsäure werden zusammen mit 50 g Thionylchlorid 16 h am Rückfluß erhitzt. Nach dem Abdestillieren des überschüssigen Thionylchlorids bleiben als Rückstand 36,8 g rohes trans,trans-4-(trans-4-Propylbicyclohexyl)-cyclohexancarbonsäurechlorid.

b) 17 g des Säurechlorids werden in 100 ml 1,4-Dioxan gelöst und diese Lösung bei Raumtemperatur zu 200 ml einer 10 %-igen wässrigen Ammoniaklösung gegeben. Das ausgefallene Carbonsäureamid wird abgesaugt und nach dem Trocknen aus einem Gemisch aus gleichen Volumenteilen Dioxan und Ethanol umkristallisiert. Ausbeute: 12,3 g trans,trans-4-(trans-4-Propylbicyclohexyl)-cyclohexancarbonsäureamid.

c) 12 g des Amids werden in 150 ml N,N-Dimethylformamid aufgeschlemmt. Der 40°C warmen Suspension werden 25 g Phosphoroxidtrichlorid beigefügt, wobei die Temperatur des Gemisches 50°C nicht überschreiten sollte. Es wird noch 3 h bei 45°C gerührt und dann in 1000 ml Eiswasser gegossen. Es wird dreimal mit 100 ml Dichlormethan extrahiert und die vereinigten organischen Phasen mit Wasser

gewaschen. Der nach dem Trocknen mit Natriumsulfat und dem Abdestillieren des Lösungsmittels verbliebene Rückstand wird aus Ethanol umkristallisiert. Ausbeute: 7,9 g trans,trans-4-(trans-4-Propylbicyclohexyl)cyclohexancarbonitril, Schmp. 95° C, Klp. 236° C.

Analog werden hergestellt:
2. trans,trans-4-(trans-4-Ethylbicyclohexyl)-cyclohexan-1-carbonitril
3. trans,trans-4-(trans-4-Butylbicyclohexyl)-cyclohexan-1-carbonitril
4. trans,trans-4-(trans-4-Pentylbicyclohexyl)-cyclohexan-1-carbonitril
5. trans,trans-4-(trans-4-Hexylbicyclohexyl)-cyclohexan-1-carbonitril
6. trans,trans-4-(trans-4-Heptylbicyclohexyl)-cyclohexan-1-carbonitril
7. trans,trans-4-(trans-4-Octylbicyclohexyl)-cyclohexan-1-carbonitril
8. trans,trans-4-(trans-4-Nonylbicyclohexyl)-cyclohexan-1-carbonitril
9. trans,trans-4-(trans-4-Decylbicyclohexyl)-cyclohexan-1-carbonitril
10. trans,trans-4-(trans-4-Ethoxybicyclohexyl)-cyclohexan-1-carbonitril
11. trans,trans-4-(trans-4-Propoxybicyclohexyl)-cyclohexan-1-carbonitril
12. trans,trans-4-(trans-4-Butoxybicyclohexyl)-cyclohexan-1-carbonitril
13. trans,trans-4-(trans-4-Pentoxybicyclohexyl)-cyclohexan-1-carbonitril
14. trans,trans-4-(trans-4-Hexoxybicyclohexyl)-cyclohexan-1-carbonitril
15. trans,trans-4-(trans-4-Heptoxybicyclohexyl)-cyclohexan-1-carbonitril
16. trans,trans-4-(trans-4-Octoxybicyclohexyl)-cyclohexan-1-carbonitril
17. trans,trans-4-(trans-4-Nonoxybicyclohexyl)-cyclohexan-1-carbonitril
18. trans,trans-4-(trans-4-Decoxybicyclohexyl)-cyclohexan-1-carbonitril

**Beispiele 19 - 108**

**Beispiel 19**

17 g des in Beispiel 1 erhaltenen Säurechlorides werden in 100 ml Methanol gelöst und die Lösung bei Raumtemperatur mit 2,5 ml Pyridin versetzt. Nach 3stgm. Rühren bei Raumtemperatur wird das überschüssige Methanol weitgehend abdestilliert und der verbliebene Rückstand mit 200 ml Wasser versetzt. Die entstandene Emulsion wird mehrmals mit je 100 ml Dichlormethan extrahiert. Aus der organischen Phase werden nach dem Umkristallisieren aus Methanol 13,2 g trans,trans-4-(trans-4-Propylbicyclohexyl)-cyclohexancarbonsäuremethylester gewonnen, Schmp. 69° C, Klp. 240° C.

Analog werden hergestellt:
20. trans,trans-4-(trans-4-Propylbicyclohexyl)-cyclohexan-carbonsäureethylester
21. trans,trans-4-(trans-4-Propylbicyclohexyl)-cyclohexan-carbonsäurepropylester
22. trans,trans-4-(trans-4-Propylbicyclohexyl)-cyclohexan-carbonsäurebutylester
23. trans,trans-4-(trans-4-Propylbicyclohexyl)-cyclohexan-carbonsäurepentylester
24. trans,trans-4-(trans-4-Propylbicyclohexyl)-cyclohexan-carbonsäurehexylester
25. trans,trans-4-(trans-4-Propylbicyclohexyl)-cyclohexan-carbonsäureheptylester
26. trans,trans-4-(trans-4-Propylbicyclohexyl)-cyclohexan-carbonsäureoctylester
27. trans,trans-4-(trans-4-Propylbicyclohexyl)-cyclohexan-carbonsäurenonylester
28. trans,trans-4-(trans-4-Propylbicyclohexyl)-cyclohexan-carbonsäuredecylester
29. trans,trans-4-(trans-4-Ethylbicyclohexyl)-cyclohexan-carbonsäuremethylester
30. trans,trans-4-(trans-4-Ethylbicyclohexyl)-cyclohexan-carbonsäureethylester
31. trans,trans-4-(trans-4-Ethylbicyclohexyl)-cyclohexan-carbonsäurepropylester
32. trans,trans-4-(trans-4-Ethylbicyclohexyl)-cyclohexan-carbonsäurebutylester
33. trans,trans-4-(trans-4-Ethylbicyclohexyl)-cyclohexan-carbonsäurepentylester
34. trans,trans-4-(trans-4-Ethylbicyclohexyl)-cyclohexan-carbonsäurehexylester
35. trans,trans-4-(trans-4-Ethylbicyclohexyl)-cyclohexan-carbonsäureheptylester
36. trans,trans-4-(trans-4-Ethylbicyclohexyl)-cyclohexan-carbonsäureoctylester
37. trans,trans-4-(trans-4-Ethylbicyclohexyl)-cyclohexan-carbonsäurenonylester
38. trans,trans-4-(trans-4-Ethylbicyclohexyl)-cyclohexan-carbonsäuredecylester
39. trans,trans-4-(trans-4-Butylbicyclohexyl)-cyclohexan-carbonsäuremethylester
40. trans,trans-4-(trans-4-Butylbicyclohexyl)-cyclohexan-carbonsäureethylester
41. trans,trans-4-(trans-4-Butylbicyclohexyl)-cyclohexan-carbonsäurepropylester
42. trans,trans-4-(trans-4-Butylbicyclohexyl)-cyclohexan-carbonsäurebutylester
43. trans,trans-4-(trans-4-Butylbicyclohexyl)-cyclohexan-carbonsäurepentylester
44. trans,trans-4-(trans-4-Butylbicyclohexyl)-cyclohexan-carbonsäurehexylester
45. trans,trans-4-(trans-4-Butylbicyclohexyl)-cyclohexan-carbonsäureheptylester
46. trans,trans-4-(trans-4-Butylbicyclohexyl)-cyclohexan-carbonsäureoctylester
47. trans,trans-4-(trans-4-Butylbicyclohexyl)-cyclohexan-carbonsäurenonylester

48. trans,trans-4-(trans-4-Butylbicyclohexyl)-cyclohexan-carbonsäuredecylester
49. trans,trans-4-(trans-4-Pentylbicyclohexyl)-cyclohexan-carbonsäuremethylester
50. trans,trans-4-(trans-4-Pentylbicyclohexyl)-cyclohexan-carbonsäureethylester
51. trans,trans-4-(trans-4-Pentylbicyclohexyl)-cyclohexan-carbonsäurepropylester
52. trans,trans-4-(trans-4-Pentylbicyclohexyl)-cyclohexan-carbonsäurebutylester
53. trans,trans-4-(trans-4-Pentylbicyclohexyl)-cyclohexan-carbonsäurepentylester
54. trans,trans-4-(trans-4-Pentylbicyclohexyl)-cyclohexan-carbonsäurehexylester
55. trans,trans-4-(trans-4-Pentylbicyclohexyl)-cyclohexan-carbonsäureheptylester
56. trans,trans-4-(trans-4-Pentylbicyclohexyl)-cyclohexan-carbonsäureoctylester
57. trans,trans-4-(trans-4-Pentylbicyclohexyl)-cyclohexan-carbonsäurenonylester
58. trans,trans-4-(trans-4-Pentylbicyclohexyl)-cyclohexan-carbonsäuredecylester
59. trans,trans-4-(trans-4-Hexylbicyclohexyl)-cyclohexan-carbonsäuremethylester
60. trans,trans-4-(trans-4-Hexylbicyclohexyl)-cyclohexan-carbonsäureethylester
61. trans,trans-4-(trans-4-Hexylbicyclohexyl)-cyclohexan-carbonsäurepropylester
62. trans,trans-4-(trans-4-Hexylbicyclohexyl)-cyclohexan-carbonsäurebutylester
63. trans,trans-4-(trans-4-Hexylbicyclohexyl)-cyclohexan-carbonsäurepentylester
64. trans,trans-4-(trans-4-Hexylbicyclohexyl)-cyclohexan-carbonsäurehexylester
65. trans,trans-4-(trans-4-Hexylbicyclohexyl)-cyclohexan-carbonsäureheptylester
66. trans,trans-4-(trans-4-Hexylbicyclohexyl)-cyclohexan-carbonsäureoctylester
67. trans,trans-4-(trans-4-Hexylbicyclohexyl)-cyclohexan-carbonsäurenonylester
68. trans,trans-4-(trans-4-Hexylbicyclohexyl)-cyclohexan-carbonsäuredecylester
69. trans,trans-4-(trans-4-Heptylbicyclohexyl)-cyclohexan-carbonsäuremethylester
70. trans,trans-4-(trans-4-Heptylbicyclohexyl)-cyclohexan-carbonsäureethylester
71. trans,trans-4-(trans-4-Heptylbicyclohexyl)-cyclohexan-carbonsäurepropylester
72. trans,trans-4-(trans-4-Heptylbicyclohexyl)-cyclohexan-carbonsäurebutylester
73. trans,trans-4-(trans-4-Heptylbicyclohexyl)-cyclohexan-carbonsäurepentylester
74. trans,trans-4-(trans-4-Heptylbicyclohexyl)-cyclohexan-carbonsäurehexylester
75. trans,trans-4-(trans-4-Heptylbicyclohexyl)-cyclohexan-carbonsäureheptylester
76. trans,trans-4-(trans-4-Heptylbicyclohexyl)-cyclohexan-carbonsäureoctylester
77. trans,trans-4-(trans-4-Heptylbicyclohexyl)-cyclohexan-carbonsäurenonylester
78. trans,trans-4-(trans-4-Heptylbicyclohexyl)-cyclohexan-carbonsäuredecylester
79. trans,trans-4-(trans-4-Octylbicyclohexyl)-cyclohexan-carbonsäuremethylester
80. trans,trans-4-(trans-4-Octylbicyclohexyl)-cyclohexan-carbonsäureethylester
81. trans,trans-4-(trans-4-Octylbicyclohexyl)-cyclohexan-carbonsäurepropylester
82. trans,trans-4-(trans-4-Octylbicyclohexyl)-cyclohexan-carbonsäurebutylester
83. trans,trans-4-(trans-4-Octylbicyclohexyl)-cyclohexan-carbonsäurepentylester
84. trans,trans-4-(trans-4-Octylbicyclohexyl)-cyclohexan-carbonsäurehexylester
85. trans,trans-4-(trans-4-Octylbicyclohexyl)-cyclohexan-carbonsäureheptylester
86. trans,trans-4-(trans-4-Octylbicyclohexyl)-cyclohexan-carbonsäureoctylester
87. trans,trans-4-(trans-4-Octylbicyclohexyl)-cyclohexan-carbonsäurenonylester
88. trans,trans-4-(trans-4-Octylbicyclohexyl)-cyclohexan-carbonsäuredecylester
89. trans,trans-4-(trans-4-Nonylbicyclohexyl)-cyclohexan-carbonsäuremethylester
90. trans,trans-4-(trans-4-Nonylbicyclohexyl)-cyclohexan-carbonsäureethylester
91. trans,trans-4-(trans-4-Nonylbicyclohexyl)-cyclohexan-carbonsäurepropylester
92. trans,trans-4-(trans-4-Nonylbicyclohexyl)-cyclohexan-carbonsäurebutylester
93. trans,trans-4-(trans-4-Nonylbicyclohexyl)-cyclohexan-carbonsäurepentylester
94. trans,trans-4-(trans-4-Nonylbicyclohexyl)-cyclohexan-carbonsäurehexylester
95. trans,trans-4-(trans-4-Nonylbicyclohexyl)-cyclohexan-carbonsäureheptylester
96. trans,trans-4-(trans-4-Nonylbicyclohexyl)-cyclohexan-carbonsäureoctylester
97. trans,trans-4-(trans-4-Nonylbicyclohexyl)-cyclohexan-carbonsäurenonylester
98. trans,trans-4-(trans-4-Nonylbicyclohexyl)-cyclohexan-carbonsäuredecylester
99. trans,trans-4-(trans-4-Decylbicyclohexyl)-cyclohexan-carbonsäuremethylester
100. trans,trans-4-(trans-4-Decylbicyclohexyl)-cyclohexan-carbonsäureethylester
101. trans,trans-4-(trans-4-Decylbicyclohexyl)-cyclohexan-carbonsäurepropylester
102. trans,trans-4-(trans-4-Decylbicyclohexyl)-cyclohexan-carbonsäurebutylester
103. trans,trans-4-(trans-4-Decylbicyclohexyl)-cyclohexan-carbonsäurepentylester
104. trans,trans-4-(trans-4-Decylbicyclohexyl)-cyclohexan-carbonsäurehexylester
105. trans,trans-4-(trans-4-Decylbicyclohexyl)-cyclohexan-carbonsäureheptylester
106. trans,trans-4-(trans-4-Decylbicyclohexyl)-cyclohexan-carbonsäureoctylester
107. trans,trans-4-(trans-4-Decylbicyclohexyl)-cyclohexan-carbonsäurenonylester
108. trans,trans-4-(trans-4-Decylbicyclohexyl)-cyclohexan-carbonsäuredecylester

**Beispiele 109 - 189**

**Beispiel 109**

trans,trans-4-(trans-4-Propylbicyclohexyl)-1-pentanoylcyclohexan

Aus 0,6 g Mg-Spänen, 10 ml Diethylether und 3,4 g n-Butylbromid wird in allgemein bekannter Weise eine Lösung von Butylmagnesiumbromid hergestellt. Zu dieser Lösung kommt unter Ausschluß von Luftfeuchtigkeit eine Suspension von 6,5 g trans,trans-4-(trans-4-Propylbicyclohexyl)-cyclohexan-1-carbonitril in 20 ml Diethylether. Das Gemisch wird 24 h unter Rückfluß gekocht und anschließend zunächst vorsichtig mit 2 ml Ethanol und dann mit 30 ml 10 %-iger Salzsäure versetzt. Die organische Phase wird abgetrennt, mit Wasser gewaschen und mit Natriumsulfat getrocknet. Der nach dem Abdampfen des Lösungsmittels verbliebene Rückstand wird 2 x aus Ethanol umkristallisiert.

Analog werden hergestellt:

110. trans,trans-4-(trans-4-Propylbicyclohexyl)-1-acetyl-cyclohexan
111. trans,trans-4-(trans-4-Propylbicyclohexyl)-1-propionyl-cyclohexan
112. trans,trans-4-(trans-4-Propylbicyclohexyl)-1-butyryl-cyclohexan
113. trans,trans-4-(trans-4-Propylbicyclohexyl)-1-hexanoyl-cyclohexan
114. trans,trans-4-(trans-4-Propylbicyclohexyl)-1-heptanoyl-cyclohexan
115. trans,trans-4-(trans-4-Propylbicyclohexyl)-1-octanoyl-cyclohexan
116. trans,trans-4-(trans-4-Propylbicyclohexyl)-1-nonanoyl-cyclohexan
117. trans,trans-4-(trans-4-Propylbicyclohexyl)-1-decanoyl-cyclohexan
118. trans,trans-4-(trans-4-Ethylbicyclohexyl)-1-acetyl-cyclohexan
119. trans,trans-4-(trans-4-Ethylbicyclohexyl)-1-propionyl-cyclohexan
120. trans,trans-4-(trans-4-Ethylbicyclohexyl)-1-butyryl-cyclohexan
121. trans,trans-4-(trans-4-Ethylbicyclohexyl)-1-pentanoyl-cyclohexan
122. trans,trans-4-(trans-4-Ethylbicyclohexyl)-1-hexanoyl-cyclohexan
123. trans,trans-4-(trans-4-Ethylbicyclohexyl)-1-heptanoyl-cyclohexan
124. trans,trans-4-(trans-4-Ethylbicyclohexyl)-1-octanoyl-cyclohexan
125. trans,trans-4-(trans-4-Ethylbicyclohexyl)-1-nonanoyl-cyclohexan
126. trans,trans-4-(trans-4-Ethylbicyclohexyl)-1-decanoyl-cyclohexan
127. trans,trans-4-(trans-4-Butylbicyclohexyl)-1-acetyl-cyclohexan
128. trans,trans-4-(trans-4-Butylbicyclohexyl)-1-propionyl-cyclohexan
129. trans,trans-4-(trans-4-Butylbicyclohexyl)-1-butyryl-cyclohexan
130. trans,trans-4-(trans-4-Butylbicyclohexyl)-1-pentanoyl-cyclohexan
131. trans,trans-4-(trans-4-Butylbicyclohexyl)-1-hexanoyl-cyclohexan
132. trans,trans-4-(trans-4-Butylbicyclohexyl)-1-heptanoyl-cyclohexan
133. trans,trans-4-(trans-4-Butylbicyclohexyl)-1-octanoyl-cyclohexan
134. trans,trans-4-(trans-4-Butylbicyclohexyl)-1-nonanoyl-cyclohexan
135. trans,trans-4-(trans-4-Butylbicyclohexyl)-1-decanoyl-cyclohexan
136. trans,trans-4-(trans-4-Pentylbicyclohexyl)-1-acetyl-cyclohexan
137. trans,trans-4-(trans-4-Pentylbicyclohexyl)-1-propionyl-cyclohexan
138. trans,trans-4-(trans-4-Pentylbicyclohexyl)-1-butyryl-cyclohexan
139. trans,trans-4-(trans-4-Pentylbicyclohexyl)-1-pentanoyl-cyclohexan
140. trans,trans-4-(trans-4-Pentylbicyclohexyl)-1-hexanoyl-cyclohexan
141. trans,trans-4-(trans-4-Pentylbicyclohexyl)-1-heptanoyl-cyclohexan
142. trans,trans-4-(trans-4-Pentylbicyclohexyl)-1-octanoyl-cyclohexan
143. trans,trans-4-(trans-4-Pentylbicyclohexyl)-1-nonanoyl-cyclohexan
144. trans,trans-4-(trans-4-Pentylbicyclohexyl)-1-decanoyl-cyclohexan
145. trans,trans-4-(trans-4-Hexylbicyclohexyl)-1-acetyl-cyclohexan
146. trans,trans-4-(trans-4-Hexylbicyclohexyl)-1-propionyl-cyclohexan
147. trans,trans-4-(trans-4-Hexylbicyclohexyl)-1-butyryl-cyclohexan
148. trans,trans-4-(trans-4-Hexylbicyclohexyl)-1-pentanoyl-cyclohexan
149. trans,trans-4-(trans-4-Hexylbicyclohexyl)-1-hexanoyl-cyclohexan
150. trans,trans-4-(trans-4-Hexylbicyclohexyl)-1-heptanoyl-cyclohexan
151. trans,trans-4-(trans-4-Hexylbicyclohexyl)-1-octanoyl-cyclohexan
152. trans,trans-4-(trans-4-Hexylbicyclohexyl)-1-nonanoyl-cyclohexan
153. trans,trans-4-(trans-4-Hexylbicyclohexyl)-1-decanoyl-cyclohexan
154. trans,trans-4-(trans-4-Heptylbicyclohexyl)-1-acetyl-cyclohexan
155. trans,trans-4-(trans-4-Heptylbicyclohexyl)-1-propionyl-cyclohexan
156. trans,trans-4-(trans-4-Heptylbicyclohexyl)-1-butyryl-cyclohexan
157. trans,trans-4-(trans-4-Heptylbicyclohexyl)-1-pentanoyl-cyclohexan
158. trans,trans-4-(trans-4-Heptylbicyclohexyl)-1-hexanoyl-cyclohexan

159. trans,trans-4-(trans-4-Heptylbicyclohexyl)-1-heptanoyl-cyclohexan
160. trans,trans-4-(trans-4-Heptylbicyclohexyl)-1-octanoyl-cyclohexan
161. trans,trans-4-(trans-4-Heptylbicyclohexyl)-1-nonanoyl-cyclohexan
162. trans,trans-4-(trans-4-Heptylbicyclohexyl)-1-decanoyl-cyclohexan
163. trans,trans-4-(trans-4-Octylbicyclohexyl)-1-acetyl-cyclohexan
164. trans,trans-4-(trans-4-Octylbicyclohexyl)-1-propionyl-cyclohexan
165. trans,trans-4-(trans-4-Octylbicyclohexyl)-1-butyryl-cyclohexan
166. trans,trans-4-(trans-4-Octylbicyclohexyl)-1-pentanoyl-cyclohexan
167. trans,trans-4-(trans-4-Octylbicyclohexyl)-1-hexanoyl-cyclohexan
168. trans,trans-4-(trans-4-Octylbicyclohexyl)-1-heptanoyl-cyclohexan
169. trans,trans-4-(trans-4-Octylbicyclohexyl)-1-octanoyl-cyclohexan
170. trans,trans-4-(trans-4-Octylbicyclohexyl)-1-nonanoyl-cyclohexan
171. trans,trans-4-(trans-4-Octylbicyclohexyl)-1-decanoyl-cyclohexan
172. trans,trans-4-(trans-4-Nonylbicyclohexyl)-1-acetyl-cyclohexan
173. trans,trans-4-(trans-4-Nonylbicyclohexyl)-1-propionyl-cyclohexan
174. trans,trans-4-(trans-4-Nonylbicyclohexyl)-1-butyryl-cyclohexan
175. trans,trans-4-(trans-4-Nonylbicyclohexyl)-1-pentanoyl-cyclohexan
176. trans,trans-4-(trans-4-Nonylbicyclohexyl)-1-hexanoyl-cyclohexan
177. trans,trans-4-(trans-4-Nonylbicyclohexyl)-1-heptanoyl-cyclohexan
178. trans,trans-4-(trans-4-Nonylbicyclohexyl)-1-octanoyl-cyclohexan
179. trans,trans-4-(trans-4-Nonylbicyclohexyl)-1-nonanoyl-cyclohexan
180. trans,trans-4-(trans-4-Nonylbicyclohexyl)-1-decanoyl-cyclohexan
181. trans,trans-4-(trans-4-Decylbicyclohexyl)-1-acetyl-cyclohexan
182. trans,trans-4-(trans-4-Decylbicyclohexyl)-1-propionyl-cyclohexan
183. trans,trans-4-(trans-4-Decylbicyclohexyl)-1-butyryl-cyclohexan
184. trans,trans-4-(trans-4-Decylbicyclohexyl)-1-pentanoyl-cyclohexan
185. trans,trans-4-(trans-4-Decylbicyclohexyl)-1-hexanoyl-cyclohexan
186. trans,trans-4-(trans-4-Decylbicyclohexyl)-1-heptanoyl-cyclohexan
187. trans,trans-4-(trans-4-Decylbicyclohexyl)-1-octanoyl-cyclohexan
188. trans,trans-4-(trans-4-Decylbicyclohexyl)-1-nonanoyl-cyclohexan
189. trans,trans-4-(trans-4-Decylbicyclohexyl)-1-decanoyl-cyclohexan

**Beispiele 190 - 199**

**Beispiel 190**

trans,trans-4-(trans-4-Propoxybicyclohexyl)-1-pentanoyl-cyclohexan

Aus 0,2 g Mg-Spänen, 5 ml Diethylether und 1,1 g n-Butylbromid wird eine Lösung von Butylmagnesiumbromid hergestellt. Zu dieser Lösung kommt unter Ausschluß von Luftfeuchtigkeit eine Suspension von 2,2 g trans,trans-4-(trans-4-Propoxybicyclohexyl)-cyclohexan-1-carbonitril in 10 ml Diethylether. Das Gemisch wird 24 h unter Rückfluß gekocht und anschließend mit 2 ml Ethanol und 30 ml 10 %-iger Salzsäure versetzt. Die organische Phase wird mit Wasser gewaschen und mit Natriumsulfat getrocknet. Der nach Abdampfen des Lösungsmittels verbliebene Rückstand wird 2mal aus Ethanol umkristallisiert.

Analog werden hergestellt:

191. trans,trans-4-(trans-4-Methoxybicyclohexyl)-1-pentanoyl-cyclohexan
192. trans,trans-4-(trans-4-Ethoxybicyclohexyl)-1-pentanoyl-cyclohexan
193. trans,trans-4-(trans-4-Butoxybicyclohexyl)-1-pentanoyl-cyclohexan
194. trans,trans-4-(trans-4-Pentoxybicyclohexyl)-1-pentanoyl-cyclohexan
195. trans,trans-4-(trans-4-Hexoxybicyclohexyl)-1-pentanoyl-cyclohexan
196. trans,trans-4-(trans-4-Heptoxybicyclohexyl)-1-pentanoyl-cyclohexan
197. trans,trans-4-(trans-4-Octoxybicyclohexyl)-1-pentanoyl-cyclohexan
198. trans,trans-4-(trans-4-Nonoxybicyclohexyl)-1-pentanoyl-cyclohexan
199. trans,trans-4-(trans-4-Decoxybicyclohexyl)-1-pentanoyl-cyclohexan

**Beispiel 200**

trans,trans-4-(trans-4-Propoxybicyclohexyl)-1-pentyl-cyclohexan

3 g des nach Beispiel 190 erhaltenen Ketons werden mit 2 g 85 %-iger Hydrazinhydratlösung, 2,5 g

pulverförmigem KOH und 15 ml Triglykol 2 h unter Rückfluß gekocht. Danach destilliert man ein Gemisch von Hydrazin und Wasser ab, bis die Temperatur im Reaktionsgemisch 195°C beträgt und hält bei dieser Temperatur, bis die Stickstoffentwicklung beendet ist. Nach dem Abkühlen wird mit dem gleichen Volumen Wasser verdünnt und mehrmals mit Dichlormethan ausgeschüttelt. Die vereinigten organischen Phasen werden mit Wasser gewaschen, mit Natriumsulfat getrocknet und durch Destillation vom Lösungsmittel befreit. Der Rückstand wird zweimal aus Isopropanol umkristallisiert.

Analog werden hergestellt:

**Beispiele 201 bis 262**

201. trans,trans-4-(trans-4-Propoxybicyclohexyl)-1-ethyl-cyclohexan
202. trans,trans-4-(trans-4-Propoxybicyclohexyl)-1-propyl-cyclohexan
203. trans,trans-4-(trans-4-Propoxybicyclohexyl)-1-butyl-cyclohexan
204. trans,trans-4-(trans-4-Propoxybicyclohexyl)-1-hexyl-cyclohexan
205. trans,trans-4-(trans-4-Propoxybicyclohexyl)-1-heptyl-cyclohexan
206. trans,trans-4-(trans-4-Propoxybicyclohexyl)-1-octyl-cyclohexan
207. trans,trans-4-(trans-4-Propoxybicyclohexyl)-1-nonyl-cyclohexan
208. trans,trans-4-(trans-4-Propoxybicyclohexyl)-1-decyl-cyclohexan
209. trans,trans-4-(trans-4-Ethoxybicyclohexyl)-1-ethyl-cyclohexan
210. trans,trans-4-(trans-4-Ethoxybicyclohexyl)-1-propyl-cyclohexan
211. trans,trans-4-(trans-4-Ethoxybicyclohexyl)-1-butyl-cyclohexan
212. trans,trans-4-(trans-4-Ethoxybicyclohexyl)-1-hexyl-cyclohexan
213. trans,trans-4-(trans-4-Ethoxybicyclohexyl)-1-heptyl-cyclohexan
214. trans,trans-4-(trans-4-Ethoxybicyclohexyl)-1-octyl-cyclohexan
215. trans,trans-4-(trans-4-Ethoxybicyclohexyl)-1-nonyl-cyclohexan
216. trans,trans-4-(trans-4-Ethoxybicyclohexyl)-1-decyl-cyclohexan
217. trans,trans-4-(trans-4-Ethoxybicyclohexyl)-1-pentyl-cyclohexan
218. trans,trans-4-(trans-4-Butoxybicyclohexyl)-1-ethyl-cyclohexan
219. trans,trans-4-(trans-4-Butoxybicyclohexyl)-1-propyl-cyclohexan
220. trans,trans-4-(trans-4-Butoxybicyclohexyl)-1-butyl-cyclohexan
221. trans,trans-4-(trans-4-Butoxybicyclohexyl)-1-hexyl-cyclohexan
222. trans,trans-4-(trans-4-Butoxybicyclohexyl)-1-heptyl-cyclohexan
223. trans,trans-4-(trans-4-Butoxybicyclohexyl)-1-octyl-cyclohexan
224. trans,trans-4-(trans-4-Butoxybicyclohexyl)-1-nonyl-cyclohexan
225. trans,trans-4-(trans-4-Butoxybicyclohexyl)-1-decyl-cyclohexan
226. trans,trans-4-(trans-4-Butoxybicyclohexyl)-1-pentyl-cyclohexan
227. trans,trans-4-(trans-4-Pentoxybicyclohexyl)-1-ethyl-cyclohexan
228. trans,trans-4-(trans-4-Pentoxybicyclohexyl)-1-propyl-cyclohexan
229. trans,trans-4-(trans-4-Pentoxybicyclohexyl)-1-butyl-cyclohexan
230. trans,trans-4-(trans-4-Pentoxybicyclohexyl)-1-hexyl-cyclohexan
231. trans,trans-4-(trans-4-Pentoxybicyclohexyl)-1-heptyl-cyclohexan
232. trans,trans-4-(trans-4-Pentoxybicyclohexyl)-1-octyl-cyclohexan
233. trans,trans-4-(trans-4-Pentoxybicyclohexyl)-1-nonyl-cyclohexan
234. trans,trans-4-(trans-4-Pentoxybicyclohexyl)-1-decyl-cyclohexan
235. trans,trans-4-(trans-4-Pentoxybicyclohexyl)-1-pentyl-cyclohexan
236. trans,trans-4-(trans-4-Hexoxybicyclohexyl)-1-ethyl-cyclohexan
237. trans,trans-4-(trans-4-Hexoxybicyclohexyl)-1-propyl-cyclohexan
238. trans,trans-4-(trans-4-Hexoxybicyclohexyl)-1-butyl-cyclohexan
239. trans,trans-4-(trans-4-Hexoxybicyclohexyl)-1-hexyl-cyclohexan
240. trans,trans-4-(trans-4-Hexoxybicyclohexyl)-1-heptyl-cyclohexan
241. trans,trans-4-(trans-4-Hexoxybicyclohexyl)-1-octyl-cyclohexan
242. trans,trans-4-(trans-4-Hexoxybicyclohexyl)-1-nonyl-cyclohexan
243. trans,trans-4-(trans-4-Hexoxybicyclohexyl)-1-decyl-cyclohexan
244. trans,trans-4-(trans-4-Hexoxybicyclohexyl)-1-pentyl-cyclohexan
245. trans,trans-4-(trans-4-Heptoxybicyclohexyl)-1-ethyl-cyclohexan
246. trans,trans-4-(trans-4-Heptoxybicyclohexyl)-1-propyl-cyclohexan
247. trans,trans-4-(trans-4-Heptoxybicyclohexyl)-1-butyl-cyclohexan
248. trans,trans-4-(trans-4-Heptoxybicyclohexyl)-1-hexyl-cyclohexan
249. trans,trans-4-(trans-4-Heptoxybicyclohexyl)-1-heptyl-cyclohexan
250. trans,trans-4-(trans-4-Heptoxybicyclohexyl)-1-octyl-cyclohexan

251. trans,trans-4-(trans-4-Heptoxybicyclohexyl)-1-nonyl-cyclohexan
252. trans,trans-4-(trans-4-Heptoxybicyclohexyl)-1-decyl-cyclohexan
253. trans,trans-4-(trans-4-Heptoxybicyclohexyl)-1-pentyl-cyclohexan
254. trans,trans-4-(trans-4-Octoxybicyclohexyl)-1-ethyl-cyclohexan
255. trans,trans-4-(trans-4-Octoxybicyclohexyl)-1-propyl-cyclohexan
256. trans,trans-4-(trans-4-Octoxybicyclohexyl)-1-butyl-cyclohexan
257. trans,trans-4-(trans-4-Octoxybicyclohexyl)-1-hexyl-cyclohexan
258. trans,trans-4-(trans-4-Octoxybicyclohexyl)-1-heptyl-cyclohexan
259. trans,trans-4-(trans-4-Octoxybicyclohexyl)-1-octyl-cyclohexan
260. trans,trans-4-(trans-4-Octoxybicyclohexyl)-1-nonyl-cyclohexan
261. trans,trans-4-(trans-4-Octoxybicyclohexyl)-1-decyl-cyclohexan
262. trans,trans-4-(trans-4-Octoxybicyclohexyl)-1-pentyl-cyclohexan

**Beispiel 263**

Zu einer Lösung von 3,3 g trans-4-(trans-trans-4-n-Pentylbicyclohex-4'-yl)-cyclohexan-1-ol (Schmp. 232°) in 200 ml Pyridin werden 2,0 g Buttersäurechlorid gegeben, die Mischung über Nacht gerührt und danach 700 ml Toluol zugegeben. Die Toluol-Phase wird nacheinander mit Salzsäure, NaOH-Lösung und Wasser gewaschen und mit $Na_2SO_4$ getrocknet. Der nach dem Abdampfen des Toluols verbliebene Rückstand wird aus Aceton umkristallisiert. Man erhält trans-4-(trans, trans-4-n-Pentylbicyclohex-4'-yl)-1-butyryloxycyclohexan.

**Beispiele 264 bis 304:**

Analog werden hergestellt:
264. trans-4-(trans,trans-4-Pentylbicyclohex-4'-yl)-1-acetoxy-cyclohexan.
265. trans-4-(trans,trans-4-Pentylbicyclohex-4'-yl)-1-propionyloxy-cyclohexan.
266. trans-4-(trans,trans-4-Pentylbicyclohex-4'-yl)-1-pentanoyloxy-cyclohexan.
267. trans-4-(trans,trans-4-Pentylbicyclohex-4'-yl)-1-hexanoyloxy-cyclohexan.
268. trans-4-(trans,trans-4-Pentylbicyclohex-4'-yl)-1-heptanoyloxy-cyclohexan.
269. trans-4-(trans,trans-4-Pentylbicyclohex-4'-yl)-1-octanoyloxy-cyclohexan.
270. trans-4-(trans,trans-4-Ethylbicyclohex-4'-yl)-1-acetoxy-cyclohexan.
271. trans-4-(trans,trans-4-Ethylbicyclohex-4'-yl)-1-propionyloxy-cyclohexan.
272. trans-4-(trans,trans-4-Ethylbicyclohex-4'-yl)-1-butyryloxy-cyclohexan.
273. trans-4-(trans,trans-4-Ethylbicyclohex-4'-yl)-1-pentanoyloxy-cyclohexan.
274. trans-4-(trans,trans-4-Ethylbicyclohex-4'-yl)-1-hexanoyloxy-cyclohexan.
275. trans-4-(trans,trans-4-Ethylbicyclohex-4'-yl)-1-heptanoyloxy-cyclohexan.
276. trans-4-(trans,trans-4-Ethylbicyclohex-4'-yl)-1-octanoyloxy-cyclohexan.
277. trans-4-(trans,trans-4-Propylbicyclohex-4'-yl)-1-acetoxy-cyclohexan.
278. trans-4-(trans,trans-4-Propylbicyclohex-4'-yl)-1-propionyloxy-cyclohexan.
279. trans-4-(trans,trans-4-Propylbicyclohex-4'-yl)-1-butyryloxy-cyclohexan.
280. trans-4-(trans,trans-4-Propylbicyclohex-4'-yl)-1-pentanoyloxy-cyclohexan.
281. trans-4-(trans,trans-4-Propylbicyclohex-4'-yl)-1-hexanoyloxy-cyclohexan.
282. trans-4-(trans,trans-4-Propylbicyclohex-4'-yl)-1-heptanoyloxy-cyclohexan.
283. trans-4-(trans,trans-4-Propylbicyclohex-4'-yl)-1-octanoyloxy-cyclohexan.
284. trans-4-(trans,trans-4-Butylbicyclohex-4'-yl)-1-acetoxy-cyclohexan.
285. trans-4-(trans,trans-4-Butylbicyclohex-4'-yl)-1-propionyloxy-cyclohexan.
286. trans-4-(trans,trans-4-Butylbicyclohex-4'-yl)-1-butyryloxy-cyclohexan.
287. trans-4-(trans,trans-4-Butylbicyclohex-4'-yl)-1-pentanoyloxy-cyclohexan.
288. trans-4-(trans,trans-4-Butylbicyclohex-4'-yl)-1-hexanoyloxy-cyclohexan.
289. trans-4-(trans,trans-4-Butylbicyclohex-4'-yl)-1-heptanoyloxy-cyclohexan.
290. trans-4-(trans,trans-4-Butylbicyclohex-4'-yl)-1-octanoyloxy-cyclohexan.
291. trans-4-(trans,trans-4-Hexylbicyclohex-4'-yl)-1-acetoxy-cyclohexan.
292. trans-4-(trans,trans-4-Hexylbicyclohex-4'-yl)-1-propionyloxy-cyclohexan.
293. trans-4-(trans,trans-4-Hexylbicyclohex-4'-yl)-1-butyryloxy-cyclohexan.
294. trans-4-(trans,trans-4-Hexylbicyclohex-4'-yl)-1-pentanoyloxy-cyclohexan.
295. trans-4-(trans,trans-4-Hexylbicyclohex-4'-yl)-1-hexanoyloxy-cyclohexan.
296. trans-4-(trans,trans-4-Hexylbicyclohex-4'-yl)-1-heptanoyloxy-cyclohexan.
297. trans-4-(trans,trans-4-Hexylbicyclohex-4'-yl)-1-octanoyloxy-cyclohexan.
298. trans-4-(trans,trans-4-Heptylbicyclohex-4'-yl)-1-acetoxy-cyclohexan.
299. trans-4-(trans,trans-4-Heptylbicyclohex-4'-yl)-1-propionyloxy-cyclohexan.
300. trans-4-(trans,trans-4-Heptylbicyclohex-4'-yl)-1-butyryloxy-cyclohexan.

301. trans-4-(trans,trans-4-Heptylbicyclohex-4'-yl)-1-pentanoyloxy-cyclohexan.
302. trans-4-(trans,trans-4-Heptylbicyclohex-4'-yl)-1-hexanoyloxy-cyclohexan.
303. trans-4-(trans,trans-4-Heptylbicyclohex-4'-yl)-1-heptanoyloxy-cyclohexan.
304. trans-4-(trans,trans-4-Heptylbicyclohex-4'-yl)-1-octanoyloxy-cyclohexan.

**Beispiel 305**

Zu einer Lösung von 1,1 g trans-4-(trans,trans-4-n-Pentylbicyclohex-4'-yl)-cyclohexan-1-ol in 250 ml THF gibt man 0,5 g 35 %-ige KH-Dispersion und rührt zwei Stunden unter Rückfluß. Dann wird 0,6 g n-Jodbutan zugegeben und zwei Stunden bei Raumtemperatur gerührt. Überschüssiges Kaliumhydrid wird mit Wasser vernichtet. Man extrahiert mit Methylenchlorid, trocknet mit $Na_2SO_4$ und dampft das Lösungsmittel ab. Der verbliebene Rückstand wird chromatographisch aufgearbeitet (Kieselgel; Ether/Petrolether = 1 : 25). Man erhält trans-4-(trans,trans-4-n-Pentylbicyclohex-4'-yl)-1-n-butoxycyclohexan.

Die anderen homologen trans-4-(trans,trans-4-n-Alkyl-bicyclohex-4'-yl)-1-n-alkoxy-cyclohexane werden aus der entsprechenden Hydroxyverbindung und dem entsprechenden n-Alkylhalogenid entsprechend Beispiel 305 erhalten.

**Beispiel 306**

Aus cis-4-[trans-4-(trans-4-n-Propylcyclohexyl)-cyclohexyl]-cyclohexancarbonsäure (F. 206°, K. 270°) erhält man analog Beispiel 1 cis-4-[trans-4-(trans-4-n-Propylcyclohexyl)-cyclohexyl]-cyclohexancarbonitril, F. 102°, K. 182°.

Analog werden hergestellt:
cis-4-[trans-4-(trans-4-Ethylcyclohexyl)-cyclohexyl]-cyclohexancarbonitril
cis-4-[trans-4-(trans-4-Butylcyclohexyl)-cyclohexyl]-cyclohexancarbonitril
cis-4-[trans-4-(trans-4-Pentylcyclohexyl)-cyclohexyl]-cyclohexancarbonitril
cis-4-[trans-4-(trans-4-Hexylcyclohexyl)-cyclohexyl]-cyclohexancarbonitril
cis-4-[trans-4-(trans-4-Heptylcyclohexyl)-cyclohexyl]-cyclohexancarbonitril

**Beispiel 307**

Aus cis-4-[trans-4-(trans-4-n-Propylcyclohexyl)-cyclohexyl]-cyclohexancarbonsäure (F. 206°, K. 270°) erhält man analog Beispiel 2 cis-4-[trans-4-(trans-4-n-Propylcyclohexyl)-cyclohexyl]-cyclohexancarbonsäuremethylester, F. 119°, K. 131°.

Analog werden hergestellt:
cis-4-[trans-4-(trans-4-Ethylcyclohexyl)-cyclohexyl]-cyclohexancarbonsäureethylester
cis-4-[trans-4-(trans-4-Ethylcyclohexyl)-cyclohexyl]-cyclohexancarbonsäurepropylester
cis-4-[trans-4-(trans-4-Ethylcyclohexyl)-cyclohexyl]-cyclohexancarbonsäurebutylester
cis-4-[trans-4-(trans-4-Ethylcyclohexyl)-cyclohexyl]-cyclohexancarbonsäurepentylester.

Die folgenden Beispiele betreffen erfindungsgemäße flüssigkristalline Phasen.

**Beispiel A**

Man stellt eine Mischung her aus (Gew.-%)
2 % trans-4-(trans,trans-4-Pentylbicyclohex-4'-yl)-1-butyryloxycyclohexan
22 % p-trans-4-Propylcyclohexyl-benzonitril
36 % p-trans-4-Pentylcyclohexyl-benzonitril
25 % p-trans-4-Heptylcyclohexyl-benzonitril
15 % 4'-(trans-4-Pentylcyclohexyl)-biphenyl-4-carbonitril.

Diese Mischung zeigt einen Schmelzpunkt von -4° C und einen Klärpunkt von 71° C.

**Beispiel B**

Man stellt eine Mischung her aus (Gew.-%)
16 % trans-4-(trans,trans-4-Pentylbicyclohex-4'-yl)-1-ethoxycyclohexan
12 % trans-4-(trans,trans-4-Pentylbicyclohex-4'-yl)-1-butoxycyclohexan
17 % p-trans-4-Propylcyclohexyl-benzonitril
23 % p-trans-4-Pentylcyclohexyl-benzonitril
22 % 4-Ethyl-4'-(trans-4-pentylcyclohexyl)-biphenyl und
10 % 4-(trans-4-Pentylcyclohexyl)-4'-(trans-4-propylcyclohexyl)-biphenyl.

**Beispiel C**

Eine Mischung aus (Gew.-%)
26 % trans,trans-4-Ethylcyclohexyl-cyclohexan-4'-carbonitril
17 % trans,trans-4-Propylcyclohexyl-cyclohexan-4'-carbonitril
21 % trans,trans-4-Butylcyclohexyl-cyclohexan-4'-carbonitril
26 % trans,trans-4-Heptylcyclohexyl-cyclohexan-4'-carbonitril
10 % trans,trans-4-(trans-4-Pentylbicyclohexyl)-cyclohexan-1-carbonitril
hat einen Klp. von 87° C.

**Beispiel D**

Eine Mischung aus (Gew.-%)
30 % r-1-Cyan-cis-4-(trans-4-pentylcyclohexyl)-1-pentylcyclohexan
27 % r-1-Cyan-cis-4-(trans-4-butylcyclohexyl)-1-heptylcyclohexan
28 % r-1-Cyan-cis-4-(trans-4-pentylcyclohexyl)-1-heptylcyclohexan
15 % trans,trans-4-(trans-4-Pentylbicyclohexyl)-cyclohexan-carbonsäureethylester
hat einen Klp. von 92 °C.

**Patentansprüche** für die Vertragsstaaten: FR, IT

1. Tercyclohexyle der Formel I

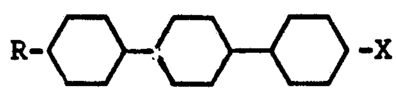

I

worin
R eine Alkylgruppe mit 1 - 12 C-Atomen, worin eine oder zwei nicht benachbarte $CH_2$-Gruppen durch 0 ersetzt sein können,
X -CN, -COOR¹, -OOCR¹, -COR¹ oder R und
R¹ eine Alkylgruppe mit 1 - 12 C-Atomen bedeuten, mit der Maßgabe, daß im Falle X = R¹ in der Alkylgruppe R mindestens eine $CH_2$-Gruppe durch 0 ersetzt ist.

2. Verfahren zur Herstellung von Tercyclohexylen der Formel I nach Anspruch 1, dadurch gekennzeichnet, daß man zur Herstellung von Carbonitrilen der Formel I (X = CN) die entsprechenden Tercyclohexylcarbonsäuren (X = COOH) oder eines ihrer reaktionsfähigen Derivate in die entsprechenden Säureamide überführt und diese dehydratisiert, oder aber ein entsprechendes Säurechlorid mit Sulfamid umsetzt, oder daß man zur Herstellung von Carbonsäureestern der Formel I (X = -COOR¹) die entsprechenden Carbonsäureverbindungen oder eines ihrer reaktionsfähigen Derivate mit einem entsprechenden Alkohol oder einem seiner reaktionsfähigen Derivate umsetzt,

oder daß man zur Herstellung von Acylverbindungen der Formel I (X = -$COR_1$) die entsprechenden Carbonsäureverbindungen oder eines ihrer reaktionsfähigen Derivate in die entsprechenden Nitrile überführt und diese mit einer entsprechenden Grignard-Verbindung umsetzt,

oder daß man zur Herstellung der Alkoxy-/Alkyl-Verbindungen (X = R¹) die entsprechenden Carbonsäureverbindungen oder eines ihrer reaktionsfähigen Derivate in die entsprechenden Ketoverbindungen (X = $COR_1$) überführt und diese reduziert,

oder daß man eine Verbindung, die sonst der Formel I entspricht, aber an Stelle von H-Atomen eine oder mehrere reduzierbare Gruppen und/oder C-C-Bindungen enthält, mit einem reduzierenden Mittel behandelt,

oder daß man zur Herstellung von Tercyclohexylen der Formel I'

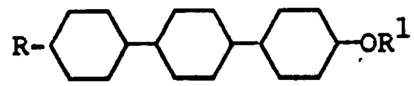

I'

worin

R eine Alkylgruppe mit 1 - 12 C-Atomen, worin eine oder zwei nicht benachbarte $CH_2$-Gruppen durch 0 ersetzt sein können, und

$R^1$ eine Alkylgruppe mit 1 - 12 C-Atomen bedeutet, dadurch gekennzeichnet, daß man die entsprechenden Tercyclohexanole ($R^1$ = H) oder eines ihrer reaktionsfähigen Derivate verethert,

oder daß man zur Herstellung von Alkanoyloxyderivaten der Formel I (X = $-OOCR^1$) die entsprechenden Tercyclohexanole (X = OH) oder eines ihrer reaktionsfähigen Derivate mit einer entsprechenden Carbonsäure oder einem ihrer reaktionsfähigen Derivate umsetzt.

3. Verwendung der Verbindungen der Formel I nach Anspruch 1 als Komponenten flüssigkristalliner Phasen.

4. Flüssigkristalline Phase mit mindestens zwei flüssigkristallinen Komponenten, dadurch gekennzeichnet, daß mindestens eine Komponente eine Verbindung der Formel I nach Anspruch 1 ist.

5. Flüssigkristallanzeigeelement, dadurch gekennzeichnet, daß es eine Phase nach Anspruch 4 enthält.

6. Elektrooptisches Anzeigeelement, dadurch gekennzeichnet, daß es als Dielektrikum eine Phase nach Anspruch 4 enthält.

**Patentansprüche** für den Vertragsstaat: DE

1. Tercyclohexyle der Formel I

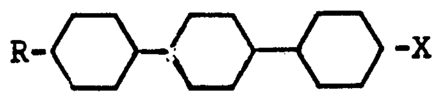

I

worin

R eine Alkylgruppe mit 1 - 12 C-Atomen, worin eine oder zwei nicht benachbarte $CH_2$-Gruppen durch 0 ersetzt sein können,

X $-COOR^1$, $-OOCR^1$, $-COR^1$ oder $R^1$ und

$R^1$ eine Alkylgruppe mit 1 - 12 C-Atomen bedeuten, mit der Maßgabe, daß im Falle X = $R^1$ in der Alkylgruppe R mindestens eine $CH_2$-Gruppe durch 0 ersetzt ist.

2. Verfahren zur Herstellung von Tercyclohexylen der Formel I nach Anspruch 1, dadurch gekennzeichnet, daß man zur Herstellung von Carbonsäureestern der Formel I (X = $-COCR^1$) die entsprechenden Carbonsäureverbindungen oder eines ihrer reaktionsfähigen Derivate mit einem entsprechenden Alkohol oder einem seiner reaktionsfähigen Derivate umsetzt,

oder daß man zur Herstellung von Acylverbindungen der Formel I (X = $-COR_1$) die entsprechenden Carbonsäureverbindungen oder eines ihrer reaktionsfähigen Derivate in die entsprechenden Nitrile überführt und diese mit einer entsprechenden Grignard-Verbindung umsetzt,

oder daß man zur Herstellung der Alkoxy-/Alkyl-Verbindungen (X = $R^1$) die entsprechenden Carbonsäureverbindungen oder eines ihrer reaktionsfähigen Derivate in die entsprechenden Ketoverbindungen (X = $COR^1$) überführt und diese reduziert,

oder daß man eine Verbindung, die sonst der Formel I entspricht, aber an Stelle von H-Atomen eine oder mehrere reduzierbare Gruppen und/oder C-C-Bindungen enthält, mit einem reduzierenden Mittel behandelt,

oder daß man zur Herstellung von Tercyclohexylen der Formel I'

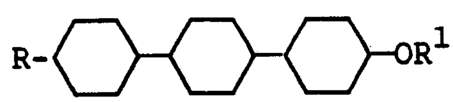

I'

worin

R eine Alkylgruppe mit 1 - 12 C-Atomen, worin eine oder zwei nicht benachbarte $CH_2$-Gruppen durch 0 ersetzt sein können, und

$R^1$ eine Alkylgruppe mit 1 - 12 C-Atomen bedeutet, dadurch gekennzeichnet, daß man die entsprechenden Tercyclohexanole ($R^1$ = H) oder eines ihrer reaktionsfähigen Derivate verethert,

oder daß man zur Herstellung von Alkanoyloxyderivaten der Formel I (X = $-OOCR^1$) die entsprechenden Tercyclohexanole (X = OH) oder eines ihrer reaktionsfähigen Derivate mit einer entsprechenden Carbonsäure oder einem ihrer reaktionsfähigen Derivate umsetzt.

3. Verwendung der Verbindungen der Formel I nach Anspruch 1 als Komponenten flüssigkristalliner Phasen.

4. Flüssigkristalline Phase mit mindestens zwei flüssigkristallinen Komponenten, dadurch gekennzeichnet, daß mindestens eine Komponente eine Verbindung der Formel I nach Anspruch 1 ist.

5. Flüssigkristallanzeigeelement, dadurch gekennzeichnet, daß es eine Phase nach Anspruch 4 enthält.

6. Elektrooptisches Anzeigeelement, dadurch gekennzeichnet, daß es als Dielektrikum eine Phase nach Anspruch 4 enthält.

**Claims** for the Contracting States: FR, IT

1. Tercyclohexyls of the formula I

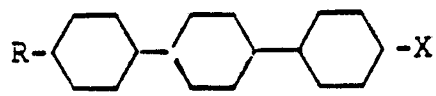

I

in which

R is an alkyl group having 1 - 12 C atoms, in which one or two non-adjacent $CH_2$ groups can be replaced by 0,

X is -CN, -COOR[1], -OOCR[1], -COR[1] or R[1], and

R[1] is an alkyl group having 1 - 12 C atoms, with the proviso that in the case where X = R[1] at least one $CH_2$ group in the alkyl group R is replaced by 0.

2. A process for the preparation of tercyclohexyls of the formula I according to Claim 1, characterised in that for the preparation of carbonitriles of the formula I (X = CN) the corresponding tercyclohexylcarboxylic acids (X = COOH), or one of their reactive derivatives, are converted into the corresponding amides, and the latter are dehydrated, or an appropriate acid chloride is reacted with sulfamide, or in that for the preparation of carboxylic esters of the formula I (X = -COOR[1]) the corresponding carboxylic acid compounds, or one of their reactive derivatives, are reacted with an appropriate alcohol or one of its reactive derivatives, or in that for the preparation of acyl compounds of the formula I (X = -$COR_1$) the corresponding carboxylic acid compounds, or one of their reactive derivatives, are converted into the corresponding nitriles, and the latter are reacted with an appropriate Grignard compound, or in that for the preparation of the alkoxy/alkyl compounds (X = R[1]) the corresponding carboxylic acid compounds, or one of their reactive derivatives, are converted into the corresponding keto compounds (X = COR[1]) and the latter are reduced, or in that a compound which corresponds to formula I apart from containing, in place of H atoms, one or more reducible groups and/or C-C bonds, is treated with a reducing agent, or in that for the preparation of tercyclohexyls of the formula I'

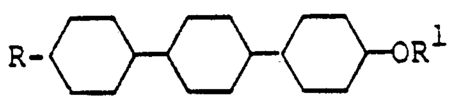

I'

in which

R is an alkyl group having 1 - 12 C atoms, in which one or two non-adjacent $CH_2$ groups can be replaced by 0, and

R[1] is an alkyl group having 1 - 12 C atoms, characterised in that the corresponding tercyclohexanols (R[1] = H), or one of their reactive derivatives, are etherified, or in that for the preparation of alkanoyloxy derivatives of the formula I (X = -OOCR[1]) the corresponding tercyclohexanols (X = OH), or one of their reactive derivatives, are reacted with an appropriate carboxylic acid or one of its reactive derivatives.

3. Use of the compounds of the formula I according to Claim 1 as components in liquid-crystalline phases.

4. Liquid-crystalline phase having at least two liquid-crystalline components, characterised in that at least one component is a compound of the formula I according to Claim 1.

5. Liquid-crystal display element, characterised in that it contains a phase according to Claim 4.

6. Electrooptical display element, characterised in that it contains as dielectric a phase according to Claim 4.

**Claims** for the Contracting State: DE

1. Tercyclohexyls of the formula I

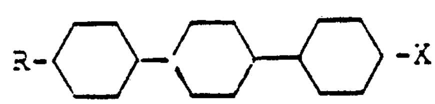

I

in which

R is an alkyl group having 1 - 12 C atoms in which one or two non-adjacent $CH_2$ groups can be replaced by 0,

X is -COOR[1], -OOCR[1], -COR[1] or R[1], and

R[1] is an alkyl group having 1 - 12 C atoms, with the proviso that in the case where X = R[1] at lesat one $CH_2$ group in the alkyl group R is replaced by 0.

2. A process for the preparation of tercyclohexyls of the formula I according to Claim 1, characterised in that

for the preparation of carboxylic esters of the formula I (X = $-COOR^1$) the corresponding carboxylic acid compounds, or one of their reactive derivatives, are reacted with an appropriate alcohol or one of its reactive derivatives, or in that for the preparation of acyl compounds of the formula I (X = $-COR_1$) the corresponding carboxylic acid compounds, or one of their reactive derivatives, are converted into the corresponding nitriles, and the latter are reacted with an appropriate Grignard compound, or in that for the preparation of the alkoxy/alkyl compounds (X = $R^1$) the corresponding carboxylic acid compounds, or one of their reactive derivatives, are converted into the corresponding keto compounds (X = $COR^1$) and the latter are reduced, or in that a compound which corresponds to formula I apart from containing, in place of H atoms, one or more reducible groups and/or C-C bonds, is treated with a reducing agent, or in that for the preparation of tercyclohexyls of the formula I'

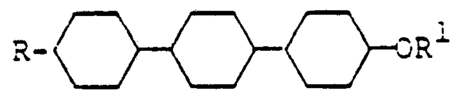

I'

in which

R is an alkyl group having 1 - 12 C atoms, in which one or two non-adjacent $CH_2$ groups can be replaced by 0, and

$R^1$ is an alkyl group having 1 - 12 C atoms, characterised in that the corresponding tercyclohexanols ($R^1$ = H), or one of their reactive derivatives, are etherified, or in that for the preparation of alkanoyloxy derivatives of the formula I (X = $-OOCR^1$) the corresponding tercyclohexanols (X = OH), or one of their reactive derivatives, are reacted with an appropriate carboxylic acid or one of its reactive derivatives.

3. Use of the compounds of the formula I according to Claim 1 as components in liquid-crystalline phases.

4. Liquid-crystalline phase having at least two liquid-crystalline components, characterised in that at least one component is a compound of the formula I according to Claim 1.

5. Liquid-crystal display element, characterised in that it contains a phase according to Claim 4.

6. Electrooptical display element, characterised in that it contains as dielectric a phase according to Claim 4.

**Revendications** pour les Etats contractants: FR, IT.

1. Tercyclohexyles de formule I

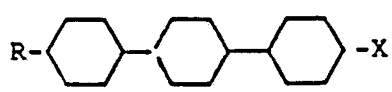

I

où

R représente un groupe alcoyle en $C_{1-12}$, où un ou 2 groupes $CH_2$ non voisins peuvent être remplacés par 0,

X représente -CN, $-COOR^1$, $OOCR^1$, $-COR^1$ ou $R^1$ et

$R^1$ représente un groupe alcoyle en $C_{1-12}$ avec cette précision que dans le cas où X = $R^1$ dans le groupe alcoyle R au moins un groupe $CH_2$ est remplacé par 0.

2. Procedé de préparation de tercyclohexylènes de formule I selon la revendication 1, caractérisé en ce que pour préparer des carbonitriles de formule I (X = CN) on transforme les acides tercyclohexylcarboxyliques correspondants (X = COOH) ou un de leurs dérivés réactifs en les amides d'acide correspondants et on déshydrate ceux-ci, ou encore en faisant réagir un chlorure d'acide correspondant avec un sulfamide, ou en ce que pour préparer des esters d'acides carboxyliques de formule I (X = $-COOR^1$) on fait réagir les composés d'acides carboxyliques correspondants ou un de leurs dérivés réactifs avec un alcool correspondant ou un de ses dérivés réactifs,

ou en ce que pour préparer des composés acyles de formule I (X = $-COR^1$) on transforme les composés d'acide carboxylique correspondants ou un de leurs dérivés réactifs en les nitriles correspondants et on fait réagir ceux-ci avec un composé de Grignard correspondant,

ou en ce que pour préparer les composés alcoxy-alcoyles (X = $R^1$) on transforme les composés d'acides carboxyliques correspondants ou un de leurs dérivés réactifs en les composés céto correspondants (X = $COR^1$) et on réduit ceux-ci,

ou en ce qu'on traite un composé qui sinon correspond à la formule I mais contient à la place d'atomes de H un ou plusieurs groupes réductibles et/ou liaisons C-C, avec un agent réducteur,

ou en ce que pour préparer du tercyclohexylène de formule I'

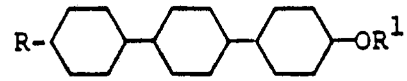

I'

où

R représente un groupe alcoyle en $C_{1-12}$, où un ou 2 groupes $CH_2$ non voisins peuvent être remplacés par 0,

et

$R^1$ représente un groupe alcoyle en $C_{1-12}$, caractérisé en ce qu'on éthérifie les tercyclohexanols correspondants ($R^1$ = H) ou un de leurs dérivés réactifs, ou en ce que pour préparer des dérivés alcanoyloxy de formule I (X = $-OOCR^1$) on fait réagir les tercyclohexanols correspondants (X = OH) ou un de leurs dérivés réactifs avec un acide carboxylique correspondant ou un de ses dérivés réactifs.

3. Application des composés de formule I selon la revendication 1 comme composants de phases à cristaux liquides.

4. Phases à cristaux liquides avec au moins deux composants à cristaux liquides caractérisées en ce qu'au moins un composant est un composé de formule I selon la revendication 1.

5. Elément d'affichage à cristaux liquides, caractérisé en ce qu'il contient une phase selon la revendication 4.

6. Elément d'affichage électro-optique, caractérisé en ce qu'il contient comme diélectrique une phase selon la revendication 4.

**Revendications** pour l'Etat Contractant: DE

1. Tercyclohexyle de formule I

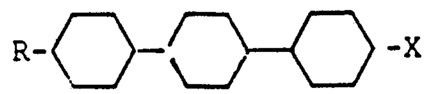

I

où

R représente un groupe alcoyle en $C_{1-12}$, où un ou 2 groupes $CH_2$ non voisins peuvent être remplacés par 0,

X représente $-COOR^1$, $OOCR^1$, $-COR^1$ ou $R^1$ et

$R^1$ représente un groupe alcoyle en $C_{1-12}$ avec cette précision que dans le cas où X = $R^1$ dans le groupe alcoyle R au moins un groupe $CH_2$ est remplacé par 0.

2. Procédé de préparation de tercyclohexylènes de formule I selon la revendication 1 caractérisé en ce que pour préparer des esters d'acides carboxyliques de formule I (X = $-COOR^1$) on fait réagir les composés d'acides carboxyliques correspondants ou un de leurs dérivés réactifs avec un alcool correspondant ou un de ses dérivés réactifs,

ou en ce que pour préparer des composés acyles de formule I (X = $-COR^1$) on transforme les composés d'acide carboxylique correspondants ou un de leurs dérivés réactifs en les nitriles correspondants et on fait réagir ceux-ci avec un composé de Grignard correspondant,

ou en ce que pour préparer les composés alcoxy-alcoyles (X = $R^1$) on transforme les composés d'acides carboxyliques correspondants ou un de leurs dérivés réactifs en les composés céto correspondants (X = $COR^1$) et on réduit ceux-ci,

ou en ce qu'on traite un composé qui sinon correspond à la formule I mais contient à la place d'atomes de H un ou plusieurs groupes réductibles et/ou liaisons C-C, avec un agent réducteur,

ou en ce que pour préparer du tercyclohexylène de formule I,

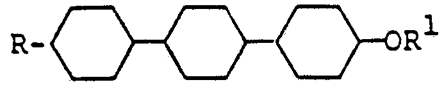

I'

ou

R représente un groupe alcoyle en $C_{1-12}$, où un ou 2 groupes $CH_2$ non voisins peuvent être remplacés par 0,

et

$R^1$ représente un groupe alcoyle en $C_{1-12}$, caractérisé en ce qu'on éthérifie les tercyclohexanols correspondants ($R^1$ = H) ou un de leurs dérivés réactifs, ou en ce que pour préparer des dérivé alcanoyloxy de formule I (X = $-OOCR^1$) on fait réagir les tercyclohexanols correspondant (X = OH) ou

un de leurs dérivés réactifs avec un acide carboxylique correspondant ou un de ses dérivés réactifs.

3. Application des composés de formule I selon la revendication 1 comme composants de phases à cristaux liquides.

4. Phases à cristaux liquides avec au moins deux composants à cristaux liquides caractérisées en ce qu'au moins un composant est un composé de formule I selon la revendication 1.

5. Elément d'affichage à cristaux liquides, caractérisé en ce qu'il contient une phase selon la revendication 4.

6. Elément d'affichage électro-optique, caractérisé en ce qu'il contient comme diélectrique une phase selon la revendication 4.